Europäisches Patentamt

European Patent Office

Office européen des brevets

(11) Veröffentlichungsnummer : **0 071 063**
**B1**

(12) **EUROPÄISCHE PATENTSCHRIFT**

(45) Veröffentlichungstag der Patentschrift :
**11.12.85**

(51) Int. Cl.⁴ : **A 61 L 15/00**

(21) Anmeldenummer : **82106196.7**

(22) Anmeldetag : **10.07.82**

(54) **Absorptionsmittel für Blut und seröse Körperflüssigkeiten.**

(30) Priorität : **16.07.81 DE 3128100**

(43) Veröffentlichungstag der Anmeldung :
**09.02.83 Patentblatt 83/06**

(45) Bekanntmachung des Hinweises auf die Patenterteilung : **11.12.85 Patentblatt 85/50**

(84) Benannte Vertragsstaaten :
**AT BE CH DE FR GB IT LI LU NL SE**

(56) Entgegenhaltungen :
**DE-A- 2 264 027**
**DE-A- 2 609 144**
**FR-A- 2 215 230**
**FR-A- 2 260 961**
**FR-A- 2 284 706**
**FR-A- 2 331 603**
**US-A- 4 055 184**
**Handbook of Chemistry and Physics, 52 Auflage, The Chemical Rubber Co., Ohio, USA (1971), S. B62 und B78**
**Ullmanns Encyklopädie der Technischen Chemie, Verlag Chemie 1979, Bd. 18 S. 334**
**Römpps Chemie Lexikon 8. Aufl. S. 572**

(73) Patentinhaber : **Chemische Fabrik Stockhausen GmbH**
**Bäkerpfad 25**
**D-4150 Krefeld (DE)**

(72) Erfinder : **Chmelir, Miroslav, Dr., Dipl.-Chem.**
**Grönkesdyk 36**
**D-4150 Krefeld (DE)**
Erfinder : **Dahmen, Kurt, Dr., Dipl.-Chem.**
**von-Velsen-Strasse 6**
**D-4050 Mönchengladbach (DE)**
Erfinder : **Hoffmann, Georg, Dr., rer. nat.**
**Westwall 165**
**D-4150 Krefeld (DE)**
Erfinder : **Werner, Georg**
**Dresdner Strasse 7**
**D-4154 Tönisvorst 1 (DE)**

(74) Vertreter : **Klöpsch, Gerald, Dr.-Ing.**
**An Gross St. Martin 6**
**D-5000 Köln 1 (DE)**

**Beschreibung**

Die Erfindung betrifft Absorptionsmittel für Blut und andere seröse Körperflüssigkeiten, die sich zur Verwendung in absorbierenden Wegwerferzeugnissen für chirurgische oder andere medizinische Zwecke sowie für Damenbinden eignen.

In den letzten Jahren wurde eine Anzahl verschiedener Polymerisate entwickelt, die hohes Absorptionsvermögen für Wasser und Körperflüssigkeiten aufweisen. Die meisten Produkte wurden auf Stärkebasis, die z. B. Stärke-Acrylnitril-Pfropfpolymerisate (US-PS 3 997 484, 3 661 815, 4 155 888, 3 935 099), gelatinisierte Stärkederivate (DE-OS 2 702 781), Stärke-Acrylamid-Acrylamidopropansulfonsäure-Pfropfpolymerisat (US-Anm. 955 827) oder auf Celulosebasis, wie Derivate von Alkyl- oder Hydroxyalkylcellulose (JA-PS 77/125.481), Carboxymethylcellulose (BE-PS 862 130, GB-PS 1 159 949) und auf Polysaccharidbasis (DE-OS 2 650 377) hergestellt. Zu den vollsynthetischen, in zahlreichen Patenten beschriebenen Absorptionsmitteln gehören vernetzte Polymere und Copolymere auf Acryl- oder Methacrylsäurebasis (DE-OS 2 429 236, DE-OS 2 614 662, US-PS 4 018 951, US-PS 3 926 891, US-PS 4 066 583, US-PS 4 062 817, DE-OS 2 712 043) DE-OS 2 653 135, DE-OS 2 650 377, DE-OS 2 813 634) oder Maleinsäurederivate (US-PS 4 041 228).

Alle diese Produkte sind praktisch wasserunlöslich, absorbieren das vielfache ihres Gewichts an Wasser, Urin oder anderen wässrigen Lösungen, weisen aber wegen geringer Dispergierbarkeit in Blut praktisch kein Absorptionsvermögen für das Blut auf.

Beim ersten Kontakt der nach dem Stande der Technik bekannten Polymerabsorptionsmittel mit dem Blut bildet sich auf dem Bluttropfen eine Haut, die als Barriere gegen das Durchdringen des Blutes zu den Absorptionsmittelteilchen wirkt. Als Ergebnis resultieren nichtbenetzte Absorptionsmittelteilchen und ein Bluttropfen mit fester Haut, der aber innen mit flüssigem Blut gefüllt ist.

Eine teilweise Verbesserung der Blutdispergierbarkeit des Absorptionsmittels wurde nach den DE-OS 2 844 956 und EU-PS 0 009 977 dadurch erreicht, daß ein teilsynthetisches oder vollsynthetisches Absorptionsmittel in Pulverform nachträglich mit Polyethern (DE-OS 2 844 956) oder mit Fettalkoholen, Fettsäuren oder -estern (EU-PS 0 009 977), meistens gelöst in organischen Lösungsmitteln, behandelt wird.

In der FR-A 2 260 961 wird ein absorbierender Wegwerfartikel beschrieben, der aus einer weichen Umhüllung und einem absorbierenden Kern besteht. Der absorbierende Kern dieses Artikels besteht aus einer Mischung eines vernetzten Copolymeren einer Carbonsäure mit einem Acetal sowie einem alkalischen Agens. Als alkalische Agentien werden Alkali- und Erdalkalisalze wie $NaHCO_3$, $KHCO_3$ oder $MgCO_3$ genannt.

In der US-A-4 055 184 wird ein absorbierendes Kissen (z. B. eine wegwerfbare Windel) beschrieben, das als absorbierende Masse ein feinverteiltes Gemisch aus einem vollständig hydrolysierten Stärke-Polyacryl-Copolymeren in saurer Form als erster Komponente und einem nicht reizend wirkenden, ungiftigen, wasserlöslichen basischen Material als zweiter Komponente besteht, wobei das Verhältnis von basischem Material zu Copolymeren 1 : 4 bis 1 : 1 beträgt. Als basisches Material werden $NaHCO_3$, $NaHPO_4$, Natriumborat, $KHCO_3$ sowie das Dinatriumphthalat gennant. Das ebenfalls genannte Mononatriumphosphat ($NaH_2PO_4$) ist ein offensichtliches Fehlzitat, da dieses Salz, wie auch das entsprechende Monoammoniumdihydrogenphosphat in wässriger Lösung sauer reagieren. Laut Ullmann, 3.Aufl., Bd.13, S.544, beträgt der pH-Wert einer 1 %-igen wässrigen Lösung des $NaH_2PO_4$ 4,5, laut Bd.3 der pH-Wert des $NH_4H_2PO_4$ in gesättigter Lösung 3,1.

In der FR-A-2 331 603 wird ein puderförmiges Absorbensgemisch aus einem wasserquellbaren Polymeren und einem wasserunlöslichen Mineralstoff beschrieben. Als Mineralstoffe sind $SiO_2$, $CaCO_3$, $MgCO_3$, $BaCO_3$, Kaolin, Diathomeen-Erde, Feldspat, Titanoxid, Aluminiumoxid jeweils in granulierter Form mit einer Korngrösse von > 50 µm genannt.

Es wurde nun überraschenderweise gefunden, daß der Zusatz einer nicht alkalischen anorganischen oder organischen, bei Normaltemperatur als rieselförmiges Pulver vorliegenden wasserlöslichen Verbindung zum Polymerabsorptionsmittel das kapillare Fließen des Blutes durch die Masse des teilchenförmigen Absorptionsmittels beschleunigen kann. In dieser Weise erfolgt eine schnelle Verteilung des Blutes in der ganzen Masse des Absorptionsmittels, so daß das Blut schneller absorbiert werden kann.

Gegenstand der Erfindung ist ein Absorptionsmittel für Blut und andere Körperflüssigkeiten, bestehend aus zwei Komponenten A und B, wobei die Komponente A wenigstens ein wasserquellbares synthetisches oder natürliches Polymeres oder Copolymeres und die Komponente B ein bei normaler Temperatur rieselfähiges Pulver ist, sowie gegebenfalls Riechstoffen, Bindemitteln oder sonstigen, die Absorptionseigenschaften nicht beinflussenden Hilfsstoffen, das dadurch gekennzeichnet, ist, daß die Komponente B wasserlöslich ist und wenigstens aus einem nicht-alkalischen Salz einer anorganischen Säure und/oder wenigstens einem Salz einer aliphatischen organischen Säure und/oder wenigstens einer anorganischen und/oder organischen Säure und/oder wenigstens einem Derivat einer Carbonsäure und/oder einem Mono- und/oder Oligosaccharid besteht.

Als Komponente A sind sowohl die wasserquellbaren Polymeren auf der Basis von Polysacchariden, wie Cellulose, Cellulosederivate wie Carboxymethylcellulose, Alkyl- oder Hydroxyalkylcellulose, Stärke

2

und Stärkederivate und Pflanzengummi (Xanthangummi, Alginsäure) und ihre Salze als auch die Polymeren oder Copolymeren auf der Basis von (Meth-)Acrylsäure oder (Meth-)Acrylsäurederivaten geeignet, wobei es sich hierbei in erster Linie um die Homo- oder Copolymere der Acryl-, Methacryl-, Acrylamidomethylpropansulfonsäure, der Salze dieser Säuren, des Acryl- oder Methacrylamids untereinander oder mit Vinylpyrrolidon und/oder Vinylacetat handelt. Die vorstehenden Polymeren können durch einen mindestens bifunktionellen Vernetzer vernetzt sein, damit sie in Wasser nur quellbar, aber nicht löslich sind. Alle diese Polymeren werden nach bekannten Verfahren hergestellt.

Als Komponente B sind besonders geeignet die gesundheitlich unbedenklichen wasserlöslichen, nicht alkalischen Salze anorganischer und/oder Salze organischer Säuren, die bei normaler Temperatur festen, pulverförmigen, gesundheitlich unbedenklichen anorganischen Säuren und/oder organischen Mono- und/oder Polycarbonsäuren und/oder niedermolekularen Carbon- bzw. Sulfonsäuren und/oder die bei normaler Temperatur festen, gesundheitlich unbedenklichen Derivate von Carbonsäuren, und/oder Mono- bzw. Oligosaccharide. Als gesundheitlich unbedenkliche wasserlösliche Salze anorganischer Säuren werden bevorzugt die Chloride, Bromide, Jodide, Sulfate, Hydrogensulfate, Dihydrogenphosphate und/oder Nitrate, als Salze organischer aliphatischer Carbonsäuren die Salze der Essig-, Ameisen-, Adipin-, Citronen- oder Weinsäure oder auch die Salze von niedermolekularen polymeren Carbon- bzw. Sulfonsäuren mit Mol-Gewichten zwischen 300 und 100 000 g/Mol, vorzugsweise 2 000 bis 20 000 g/Mol, auf der Basis von Homo- oder Copolymerisaten ungesättigter Mono- oder Dicarbonsäuren, Sulfonsäuren, Aldehydren, Alkoholen sowie (Meth-)Acrylamid verwendet.

Geeignete wasserlösliche Salze sind die Ammonium-, Natrium-, Kalium-, Lithium-, Calcium-, Magnesium-, Zink-, Aluminium- oder Eisensalze der anorganischen oder organischen Säuren, sofern die Salze der anorganischen Säuren nicht alkalisch sind.

Es können auch die anorganischen oder organischen Säuren selbst, sofern sie bei normaler Temperatur fest, pulverförmig und wasserlöslich sind, als Komponente B verwendet werden. Geeignete anorganische Säuren sind Borsäure oder Phosphorsäure. Geeignete organische Säuren sind Mono- oder Polycarbonsäuren wie Citronen-, Wein- oder Adipinsäure oder niedermolekulare polymere Carbon- bzw. Sulfonsäuren mit Molekulargewichten zwischen 300 und 100 000 g/Mol, vorzugsweise zwischen 2 000 und 20 000 g/Mol auf der Basis von Homo- oder Copolymerisaten ungesättigter Mono- oder Dicarbonsäuren, Sulfonsäuren, Aldehyden, Alkoholen sowie (Meth-)Acrylamid.

Weiter sind geeignet die wasserlöslichen, bei Normaltemperatur festen Derivate von Carbonsäuren wie Amide oder Diamide, vorzugsweise Acetamid, Harnstoff und Harnstoff-Derivate wie Thioharnstoff, Methyl- oder Ethylharnstoff.

Schließlich sind als Komponente B auch Mono- oder Oligosaccharide, wie Glukose, Fructose, Mannose oder Saccharose geeignet.

Das Absorptionsmittel besteht aus den Komponenten A und B in einem Gewichtsverhältnis 25 bis 98 Gew.-%, vorzugsweise 50 bis 90 Gew.-%, der Komponente A, zu 2 bis 75 Gew.-%, vorzugsweise 10 bis 50 Gew.-%, der Komponente B.

Das Vermischen der beiden Komponenten A und B kann dadurch erfolgen, daß die Komponente B schon in der Monomerlösung vor der Polymerisation gelöst wird oder daß die Komponente B zu irgend einem Zeitpunkt der Herstellung in trockener oder gelöster Form zugesetzt wird.

Das erfindungsgemäße Absorptionsmittel ist aufgrund seiner Zusammensetzung zur Aufnahme und/oder Zurückhaltung von Blut und anderen Körperflüssigkeiten, besonders für den Einsatz in absorbierenden Wegwerferzeugnissen, wie Damenbinden, Tampons oder in absorbierenden Erzeugnissen für chirurgische und medizinische Verwendung geeignet.

Das erfindungsgemässe Absorptionsmittel wird, je nach Einsatzzweck in entsprechender Dosierung, meistens in oder auf eine Textil- oder Papierunterlage eingestreut und in oder auf dem Stoff durch geeignete Massnahmen fixiert.

Zusätzlich können dem erfindungsgemäßen Absorptionsmittel Riechstoffe, Bindemittel oder sonstige Hilfsstoffe, wie z. B. Desinfektionsmittel, die die Absorptionseigenschaften des Absorptionsmittels nicht beeinflussen, zugemischt werden.

Die Herstellung der Komponente A ist in den Beispielen 1 bis 6 erläutert:

### Beispiel 1

In einem Polymerisationsgefäss wurden 328 g Acrylsäure, 2,6 g N,N'-Methylenbisacrylamid in 980 g Wasser gelöst und mit 127,5 g Natriumhydrogencarbonat auf pH = 4,0 eingestellt. Bei normaler Temperatur wurden die Komponenten des Katalysatorsystems (0,36 g Azobisamidinpropandihydrochlorid, 0,73 g Kaliumpersulfat, 1,34 g Natriumpyrosulfit und 0,06 g Eisen(II)-gluconat), gelöst in 120 ml Wasser, zugegeben, wobei adiabatische Polymerisation erfolgt. Das entstandene Polymergel wurde zerkleinert, getrocknet und gemahlen.

### Beispiel 2

In einem Polymerisationsgefäss wurden 375 g Acrylsäure und 0,75 g N,N'-Methylenbisacrylamid in 850 g Wasser gelöst und mit 120 g 25 %iger Ammoniaklösung auf pH = 4,0 neutralisiert. Für die

3

Polymerisation wurde das gleiche Katalysatorsystem wie in Beispiel 1 benutzt und das entstandene Polymergel in gleicher Weise verarbeitet.

## Beispiel 3

In einem Polymerisationsgefäss wurden 140 g Acrylamid, 35,6 g Acrylsäure und 1,8 g N,N'-Methylenbisacrylamid in 550 g destilliertem Wasser gelöst und mit 10 g Natriumhydrogencarbonat auf pH = 4,0 neutralisiert. Bei normaler Temperatur wurden die einzelnen Komponenten des katalysatorsystems (0,64 g Natriumpyrosulfat, 0,36 g Kaliumpersulfat und 0,03 g Eisen(II)-gluconat), gelöst in 60 g Wasser, zugeben, womit die Polymerisation gestartet wurde. Die Aufarbeitung erfolgte wie im Beispiel 1.

## Beispiel 4

In einem Polymerisationsgefäss wurden 568 g Acrylsäure, 0,75 g Tetraallyloxiethan und 181,5 g Acrylamidopropansulfonsäure in 1 930 g Wasser gelöst und mit 256 g Natriumhydrogencarbonat auf pH = 4,5 neutralisiert. Nach Zugabe von 1,2 g Azobisamidinpropandihydrochlorid erfolgt bei normaler Temperatur durch UV-Licht eine photochemische Polymerisation. Das Polymergel wurde zerkleinert, getrocknet und gemahlen.

## Beispiel 5

In einem Polymerisationsgefäss werden 328 Methacrylsäure, 48 g Vinylpyrrolidon und 0,75 g Trimethylolpropandiallylether in 1 060 g Wasser gelöst und mit 34,6 g Natriumhydrogencarbonat auf pH = 4,2 neutralisiert. Unter Zusatz von 0,6 g Azobisamidinpropandihydrochlorid wurde photochemisch polymerisiert und das Polymergel wie in Beispiel 1 aufgearbeitet.

## Beispiel 6

In einem Polymerisationsgefäss werden 320 g Acrylsäure, 56 g Vinylpyrrolidon und 3,75 g N,N'-Methylenbisacrylamid in 862 g Wasser gelöst und mit 100 g Natriumhydrogencarbonat auf pH = 4,4 neutralisiert. Bei normaler Temperatur wurden die einzelnen Komponenten des Katalysatorsystems (0,6 g Azobisamidinpropandihydrochlorid, 1,2 g Natriumpyrosulfit und 0,6 g Kaliumpersulfat), gelöst in 150 g Wasser, zudosiert. Die Polymerisation erfolgt praktisch adiabatisch. Das entstandene Polymergel wurde zerkleinert, getrocknet und gemahlen.

## Beispiel 7

In einem Polymerisationsgefäss werden 320 g Acrylsäure, 56 g Vinylpyrrolidon, 3,75 g, N,N'-Methylenbisacrylamid und 54 g Natriumchlorid in 700 g Wasser gelöst und mit 125 g Natriumhydrogencarbonat auf pH = 4,0 neutralisiert. Bei normaler Temperatur wurde 0,6 g Azobisamidinpropandihydrochlorid zugegeben und photochemisch (UV-Licht) polymerisiert. Das entstandene Polymergel wurde zerkleinert, getrocknet und gemahlen.

## Beispiel 8.

Zu den in den Beispielen 1 bis 6 hergestellten Produkten (Komponente A) wurden die in der Tabelle 1 aufgeführten Salze (Komponente B) in Pulverform homogen zugemischt. Zur Bestimmung der Geschwindigkeit der Verteilung des Blutes im Absorptionsmittel und der festgehaltenen Menge des Blutes durch das Absorptionsmittel wurde folgende Prüfmethode angewandt :

Auf eine Filterpapiervorlage (Ø 45 mm) wurde eine Plexiglasplatte mit einem runden Ausschnitt (Ø 40 mm) gelegt, das zu prüfende Absorptionsmittel wurde in die Öffnung aufgestreut und gleichmäßig auf die ganze kreisförmige Fläche verteilt. Danach wurde in die Mitte des Kreises 0,5 ml Humanblut dosiert und die Zeit gemessen, in der der durch die kapillaren Kräfte sich bildende Blutfleck eine Grösse von 20 mm erreicht. Nach 60 Sekunden wurde die geprüfte Probe mit Filterpapier (Ø 45 mm) bedeckt, mit einem Gewicht von 500 g belastet (40 g/cm$^2$) und die Menge des durch das Absorptionsmittel absorbierten Blutes ermittelt, wobei die Menge des nichtverbrauchten Absorptionsmittel sowie die Blutmenge, die durch die Filterpapierauflage und -unterlage absorbiert wurde, berücksichtigt wurde. Die Ergebnisse sind in Tabelle 1 zusammengefasst.

In gleicher Weise wurden auch die erfindungsgemäßen Absorptionsmittel geprüft, die aus Polymerisaten auf Naturbasis (Komponente A) hergestellt wurden. Die Ergebnisse sind in Tabelle 2 zusammengefasst.

4

Tabelle 1

| Komponente A | Komponente B | Verhältnis A/B | absorbierte Blutmenge in % bezogen auf eingesetzte Blutmenge | Komponente A | Geschwindigkeit der Blutverteilung in s |
|---|---|---|---|---|---|
| Beispiel 1 | — | — | 34,0 | — | > 60 |
| " | KCl | 2 : 1 | 74,0 | 68 | 3 |
| " | KCl | 3 : 1 | 74,6 | 85 | 4 |
| " | KCl | 5 : 1 | 90,6 | 98 | 11 |
| " | KCl | 7 : 1 | 85,2 | 100 | 12 |
| " | KCl | 9 : 1 | 81,8 | 110 | 30 |
| " | KCl | 19 : 1 | 64,4 | 140 | 48 |
| " | $NH_4Cl$ | 5 : 1 | 84,6 | 83 | 3 |
| " | NaCl | 5 : 1 | 83,6 | 78 | 10 |
| " | $Na_2SO_4$ | 5 : 1 | 77,2 | 100 | 5 |
| " | KBr | 3 : 1 | 92,0 | 88 | 4 |
| " | $KHSO_4$ | 3 : 1 | 89,0 | 95 | 7 |
| " | $K_2SO_4$ | 3 : 1 | 91,0 | 105 | 4,5 |
| " | $KNO_3$ | 2 : 1 | 89 | 91 | 11 |
| " | $NaNO_3$ | 2 : 1 | 87 | 89 | 12 |
| " | $NaPO_3$ | 3 : 1 | 90 | 96 | 18 |
| " | $NH_4H_2PO_4$ | 3 : 1 | 86 | 102 | 12 |
| " | o-Phosphorsäure | 3 : 1 | 95 | 135 | 16 |
| " | m-Phosphorsäure | 3 : 1 | 81 | 77 | 24 |
| " | Borsäure | 3 : 1 | 45 | 198 | 48 |
| " | $CaCl_2$ | 3 : 1 | 79 | 78 | 30 |
| " | $NH_4Fe(SO_4)_2$ | 3 : 1 | 81 | 85 | 14 |
| " | $Ca(CH_3COO)_2$ | 3 : 1 | 91 | 110 | 6 |
| " | $Ca(H_2PO_4)_2$ | 3 : 1 | 90 | 98 | 18 |
| " | $KAl(SO_4)_2$ | 5 : 1 | 87,3 | 138 | 18 |
| " | $Al_2(SO_4)_3$ | 5 : 1 | 90,1 | 108 | 16 |
| " | $CH_3COOK$ | 5 : 1 | 82,5 | 194 | 3 |
| " | $CH_3COONa$ | 4 : 1 | 90,7 | 125 | 4 |
| " | $CH_3COONa$ | 9 : 1 | 90,0 | 147 | 5 |
| " | $(CH_3COO)_2Mg$ | 5 : 1 | 93,0 | 114 | 4 |
| " | Kaliumtartrat | 3 : 1 | 85,0 | 102 | 18 |
| " | Natriumcitrat | 3 : 1 | 91,0 | 110 | 12 |
| " | $CH_3CONH_2$ | 4 : 1 | 100,0 | 130 | 5 |
| " | Saccharose | 4 : 1 | 78,0 | 240 | 55 |
| " | Glukose | 4 : 1 | 95,9 | 195 | 15 |
| " | Citronensäure | 4 : 1 | 92,6 | 180 | 13,6 |
| " | Citronensäure/ KCl (1 : 1) | 4 : 1 | 100,0 | 120 | 8,5 |
| " | Harnstoff | 4 : 1 | 99,0 | 114 | 8,8 |
| " | Ethylharnstoff | 4 : 1 | 98,1 | 138 | 12,4 |
| Beispiel 2 | — | — | 40,1 | — | > 60 |
| " | KCl | 5 : 1 | 85,6 | 94 | 4 |
| Beispiel 3 | — | — | 45,0 | — | > 60 |
| " | NaCl | 3 : 1 | 79,0 | 96 | 45 |
| Beispiel 4 | — | — | 53,1 | — | > 60 |
| " | KCl | 5 : 1 | 93,5 | 82 | 3,5 |
| Beispiel 5 | — | — | 55,0 | — | > 60 |
| " | NaCl | 2 : 1 | 95,0 | 81 | 15 |
| Beispiel 6 | — | — | 61,5 | — | > 60 |
| " | KCl | 5 : 1 | 75,3 | 85 | 12 |
| " | NaCl | 1 : 1 | 85,5 | 90 | 2 |
| " | $NH_4Cl$ | 2 : 1 | 90,1 | 112 | 15 |
| " | Na-Polyacrylat Molgew. 4 000 g/mol | 4 : 1 | 72,0 | 190 | 12 |

# 0 071 063

(Fortsetzung)

| Komponente A | Komponente B | Verhältnis A/B | absorbierte Blutmenge in % bezogen auf eingesetzte Blutmenge | Komponente A | Geschwindigkeit der Blutverteilung in s |
|---|---|---|---|---|---|
| " | Na-Acrylat/ Acrylamid- Copolymerisat Molgew. 9 000 g/mol | 3 : 1 | 85,0 | 120 | 17 |
| " | Acrylsäure/2-Acryl- amido-2-methyl- propansulfonsäu- re-Copolymeri- sat Nasalz Molgew. 15 000 g/mol. | 3 : 1 | 91,0 | 118 | 12 |
| Beispiel 7 | NaCl | 6 : 1 | 94,5 | 96 | 3 |
| " | NaCl/KCl (1 : 1) | 7 : 3 | 95,0 | 83 | 3 |
| Polyacrylamid Molgew. $5.10^6$ g/mol | $CH_3COONa$ | 4 : 1 | 81,0 | 143 | 14,0 |
| Polyacrylamid$_6$ Molgew. $1.10^6$ g/mol | — | — | 42,7 | 94 | 35,0 |
| " | Ethylharnstoff | 3 : 1 | 70,0 | 123 | 14,0 |
| " | $CH_3COONa$ | 3 : 1 | 58,0 | 84 | 13,2 |
| Acrylamid/ Acrylsäure Copolymerisat, Molgew. $6.10^6$ g/mol | $CH_3COONa$ | 4 : 1 | 75,0 . | 134 | 14,4 |

Tabelle 2

| Komponente A | Komponente B | Verhältnis A/B | absorbierte Blutmenge in % bezogen auf eingesetzte Blutmenge | Komponente A | Geschwindigkeit der Blutverteilung in s |
|---|---|---|---|---|---|
| vernetztes Stärke Acrylsäure- Copolymerisat | — | — | 15,0 | — | > 60 |
| " | KCl | 2 : 1 | 37,0 | 180 | 60 |
| " | KCl | 1 : 1 | 55,1 | 250 | 45 |
| " | KCl | 1 : 3 | 85,5 | 380 | 13,5 |
| Carboxyme- thylcellulose | — | — | 18,0 | — | > 60 |
| " | $CH_3COONa$ | 1 : 1 | 94,9 | 180 | 14 |
| Methylhydroxy- ethylcellulose | — | — | 12 | — | > 60 |
| " | $CH_3COONa$ | 1 : 1 | 79,2 | 184 | 45 |
| Cellulose MN 100 | — | — | 24,0 | — | > 60 |
| " | $CH_3COONa$ | 1 : 1 | 88,9 | 230 | 17,9 |
| Stärke | — | — | 18,0 | — | > 60 |
| " | $CH_3COONa$ | 1 : 2 | 87,3 | ~ 150 | 45 |

**Patentansprüche**

1. Absorptionsmittel für Blut und andere Körperflüssigkeiten, bestehend aus zwei Komponenten A und

6

0 071 063

B, wobei die Komponente A wenigstens ein wasserquellbares synthetisches oder natürliches Polymeres oder Copolymeres und die Komponente B ein bei normaler Temperatur rieselfähiges Pulver ist sowie gegebenenfalls Riechstoffen, Bindemitteln oder sonstigen, die Absorptionseigenschaften nicht beinflussenden Hilfsstoffen, dadurch gekennzeichnet, dass die Komponente B wasserlöslich ist und wenigstens aus einem nicht-alkalischen Salz einer anorganischen Säure und/oder wenigstens einem Salz einer aliphatischen organischen Säure und/oder wenigstens einer anorganische und/oder organischen Säure und/oder wenigstens einem Derivat einer Carbonsäure und/oder einem Mono- und/oder Oligosaccharid besteht.

2. Absorptionsmittel nach Anspruch 1, dadurch gekennzeichnet, daß die Komponente A ein Polymeres oder ein Copolymeres auf Basis von (Meth-)Acrylsäure oder eines (Meth-)Acrylsäurederivats, vorzugsweise ein Homo- oder Copolymeres der Acryl-, Methacryl-, Acrylamidomethylpropansulfonsäure, den Salzen dieser Säuren, des Acryl- oder Methacrylamids untereinander oder mit Vinylpyrrolidon und/oder Vinylacetat ist.

3. Absorptionsmittel nach Anspruch 1, dadurch gekennzeichnet, daß die Komponente A ein Polymeres oder Copolymeres auf Basis von Polysacchariden, bevorzugt Stärkte oder Cellulose, oder deren Derivaten ist.

4. Absorptionsmittel nach einem der Ansprüche 1 bis 3, dadurch gekennzeichnet, daß die Komponente B als Salz einer anorganischen Säure ein Chlorid, Bromid, Jodid, Sulfat, Hydrogensulfat, Dihydrogenphosphat oder Nitrat enthält.

5. Absorptionsmittel nach einem der Ansprüche 1 bis 4, dadurch gekennzeichnet, daß die Komponente B ein Salz der Essig-, Ameisen-, Adipin-, Citronen- oder Weinsäure, oder ein Salz einer nieder-molekularen polymeren Carbon- bzw. Sulfonsäure mit einem Molgewicht zwischen 300 und 100 000, vorzugsweise 2 000 bis 20 000 auf des Basis von Homo- und Copolymerisaten ungesättigter Mono- oder Dicarbonsäuren, Sulfonsäuren, Aldehyden, Alkoholen sowie (Meth-)Acrylamid enthält.

6. Absorptionsmittel nach einem der Ansprüche 1 bis 5, dadurch gekennzeichnet, daß die Komponente B ein gesundheitlich unbedenkliches Ammonium-, Natrium-, Kalium-, Lithium-, Calcium-, Magnesium-, Zink-, Aluminium- oder Einsensalz einer anorganischen oder organischen Säure allein oder im Gemisch untereinander enthält.

7. Absorptionsmittel nach einem der Ansprüche 1 bis 3, dadurch gekennzeichnet, daß die Komponente B Borsäure oder Phosphorsäure, oder eine organische Mono- oder Polycarbonsäure, bevorzugt Citronen-, Wein- oder Adipinsäure, oder eine nieder-molekulare polymere Carbon- bzw. Sulfonsäure mit Molekulargewichten zwischen 300 und 100 000 g/mol, vorzugsweise zwischen 2 000 und 20 000 g/mol, auf der Basis von Homo- oder Copolymerisaten ungesättigter Mono- oder Dicarbonsäuren, Sulfonsäuren, Aldehyden, Alkoholen sowie (Meth-)Acrylamid ist.

8. Absorptionsmittel nach einem der Ansprüche 1 bis 3, dadurch gekennzeichnet, daß die Komponente B als Derivat einer Carbonsäure ein Amid oder Diamid, Harnstoff oder ein Harnstoffderivat, bevorzugt Thioharnstoff, Methyl- oder Ethylharnstoff ist.

9. Absorptionsmitel nach einem der Ansprüche 1 bis 3, dadurch gekennzeichnet, daß die Komponente B Glukose, Fructose, Mannose oder Saccharose ist.

10. Absorptionsmittel nach einem der Ansprüche 1 bis 9, dadurch gekennzeichnet, daß das Absorptionsmittel 10 bis 98 Gew.-%, vorzugsweise 50 bis 90 Gew.-% an Komponente A und 2 bis 90, vorzugsweise 10 bis 50 Gew.-% an Komponente B enthält.

11. Verwendung des Absorptionsmittels nach Ansprüchen 1 bis 10 bei der Herstellung von absorbierenden Wegwerferzeugnissen für chirurgische und andere medizinische und hygienische Zwecke.


## Claims

1. Absorbent for blood and other body liquids, containing two components A and B, whereby component A is at least a waterswellable sythetic or natural polymer or copolymer and component B is a pourable powder at normal temperatures, and further optionally containing odoriferous substances or other auxiliaries not influencing the absorption properties, characterized in that component B is watersoluble and contains at least one non-alcaline salt of an inorganic acid and/or at least one salt of an aliphatic organic acid and/or at least one inorganic and/or organic acid and/or at least a derivative of a carboxylic acid and/or a mono and/or oligo saccharide.

2. Absorbent as in Claim 1, characterized in that component A is a polymer or copolymer of (meth)acrylic acid or of a (meth)acrylic acid derivative, preferably a homo- or copolymer of the acrylic, methacrylic, acryloamidomethyl propane sulphonic acids, the salts of said acids ; of acrylic or methacrylic amides with one another of with vinyl pyrrolidone and/or vinyl acetate.

3. Absorbent as in Claim 1, characterized in that component A is a polysaccharide polymer or copolymer, preferably of starch or cellulose or the derivatives thereof.

4. Absorbent as in either one of Claims 1 to 3, characterized in that component B contains as salt of an inorganic acid a chloride, bromide, iodide, sulfate, hydrogensulfate, dihydrogen phosphate, or nitrate.

5. Absorbent as in either one of Claims 1 to 4, characterized in that component B contains a salt of

7

the acetic, formic, adipic, citric or tartaric acid, or the salt of a low-molecular polymeric carboxylic or sulphonic acid having a molecular mass ranging from 300 to 100,000, preferably 2,000 to 20,000 based on homo- and copolymerizates of unsaturated mono- or dicarboxylic acids, sulphonic acids, aldehydes, alcohols, as well as (meth)acryloamide.

6. Absorbent as in either one of Claims 1 to 5, characterized in that component B contains an ammonium, sodium, potassium, lithium, calcium, magnesium, zinc, aluminium, or iron salt, not injurious to health, of an inorganic or organic acid either alone or in a mixture of the aforegoing.

7. Absorbent as in either one of Claims 1 to 3, characterized in that component B is boric or phosphoric acid, or an organic mono- or polycarboxylic acid, preferably citric, tartaric, or adipic acid, or a low-molecular polymeric carboxylic or sulphonic acid having a molecular mass ranging from 300 to 100,000 g/mol, preferably 2,000 to 20,000 g/mol, based on homo- or copolymerizates of unsaturated mono- or dicarboxylic acids, sulphonic acids, aldehydes, alcohols, as well as (meth)acryloamide.

8. Absorbent as in either one of the Claims 1 to 3, characterized in that component B is as derivative of a carboxylic acid an amide or diamide, urea or a urea derivative, preferably thiorurea, methyl or ethyl urea.

9. Absorbent as in either one of the Claims 1 to 3, characterized in that component B is glucose, fructose, mannose or saccharose.

10. Absorbent as in either one of Claims 1 to 9, characterized in that the absorbent contains 10 to 98 weight %, preferably 50 to 90 weight % of component A and 2 to 90, preferably 10 to 50 weight % of component B.

11. Use of the absorbent as per Claims 1 to 10 for the production of absorbing throw-away products for surgical and other medical and hygienic purposes.


**Revendications**

1. Agent d'absorption du sang et d'autres liquides corporels séreux, formé de deux composantes A et B, dont la composante A est constituée d'au moins un copolymère ou polymère naturel ou synthétique, gonflable à l'eau, et la composante B d'un produit pulvérulent s'écoulant librement à la température normale, et contenant éventuellement des substances odorantes, des liants ou d'autres additifs ne diminuant pas les propriétés d'absorption, caractérisé en ce que la composante B est soluble dans l'eau et constituée d'au moins un sel non alcalin d'un acide minéral et(ou) d'au moins un sel d'un acide organique aliphatique et(ou) d'au moins un sel d'un acide minéral et(ou) organique et(ou) d'au moins un dérivé d'un acide carboxylique et(ou) d'un mono- et(ou) oligo-saccharide.

2. Agent d'absorption suivant la revendication 1, caractérisé en ce que la composante A est formée d'un polymère ou d'un copolymère à base d'acide (méthyl)acrylique, de préférence d'un homo- ou copolymère de l'acide acryl-, méthacryl- ou acrylamido-méthyl-propane-sulfonique, de sels de ces acides, d'acryl- ou de méthyacryl-amide ou de ces substances entre elles ou avec la vinyl-pyrrolidone et(ou) l'acétate de vinyle.

3. Agent d'absorption suivant la revendication 1, caractérisé en ce que la composante A est formée d'un polymère ou d'un copolymère à base de polysaccharides, de préférence un amidon ou une cellulose ou leurs dérivés.

4. Agent d'absorption suivant l'une quelconque des revendications 1 à 3, caractérisé en ce que la composante B contient comme sel d'un acide minéral un chlorure, un bromure, un iodure, un sulfate, un hydrogénosulfate, un dihydrogéno-phosphate ou un nitrate.

5. Agent d'absorption suivant l'une quelconque des revendications 1 à 4, caractérisé en ce que la composante B contient un sel de l'acide acétique, formique, adipique, citrique ou tartrique ou un sel d'un acide carboxylique ou sulfonique polymère à bas poids moléculaire, compris entre 300 et 100 000 et de préférence entre 2 000 et 20 000, à base d'homopolymères et de copolymères d'acides mono- ou di-carboxyliques insaturés ou d'acides sulfoniques, d'aldéhydes, d'alcools ou de (méth)acryl-amide.

6. Agent d'absorption suivant l'une quelconque des revendications 1 à 5, caractérisé en ce que la composante B contient, seul ou en mélange, un sel d'ammonium, de sodium, de potassium, de lithium, de calcium, de magnésium, de zinc, d'aluminium ou de fer d'un acide minéral ou organique physiologiquement compatible.

7. Agent d'absorption suivant l'une quelconque des revendications 1 à 3, caractérisé en ce que la composante B est formée d'acide borique ou d'acide phosphorique ou d'un acide organique mono- ou poly-carboxylique, de préférence d'acide citrique, d'acide tartrique ou d'acide adipique, ou d'un acide carboxylique ou sulfonique polymère à bas poids moléculaire, de l'ordre de 300 à 100 000 et de préférence de 2 000 à 20 000 g/mole, à base d'homopolymère ou de copolymères d'acides mono- ou di-carboxyliques insaturés, d'acides sulfoniques, d'aldéhydes, d'alcools ou de (méth)acryl-amide.

8. Agent d'absorption suivant l'une quelconque des revendications 1 à 3, caractérisé en ce que la composante B est un dérivé d'un acide carboxylique tel qu'un amide ou diamide, de l'urée ou un dérivé d'urée, de préférence la thio-urée ou la méthyl- ou l'éthyl-urée.

9. Agent d'absorption suivant l'une quelconque des revendications 1 à 3, caractérisé en ce que la composante B est du glucose, du fructose, du mannose ou du saccharose.

10. Agent d'absorption suivant l'une quelconque des revendications 1 à 9, caractérisé en ce qu'il est composé de 10 à 98 % et de préférence de 50 à 90 % en poids de la composante A et de 2 à 90 et de préférence de 10 à 50 % en poids de la composante B.

11. Utilisation de l'agent d'absorption suivant l'une quelconque des revendications 1 à 10 pour la production d'articles absorbants à jeter pour des fins chirurgicales ou pour d'autres fins médicales et hygiéniques.